Europäisches Patentamt

European Patent Office

Office européen des brevets

(11) Publication number: **0 457 672 A1**

(12) **EUROPEAN PATENT APPLICATION**

(21) Application number: **91401244.8**

(22) Date of filing: **14.05.91**

(51) Int. Cl.$^5$: **A61K 7/06, A61K 7/48, C11D 3/22**

(30) Priority: **16.05.90 JP 125868/90**
**18.09.90 JP 247944/90**

(43) Date of publication of application:
**21.11.91 Bulletin 91/47**

(84) Designated Contracting States:
**DE FR GB**

(71) Applicant: **SHIN-ETSU CHEMICAL CO., LTD.**
**6-1, Ohtemachi 2-chome**
**Chiyoda-ku Tokyo (JP)**

(72) Inventor: **Obara, Sakae**
**2-5-32, Nishishiro-cho**
**Joetsu-shi, Niigata-ken (JP)**

Inventor: **Tanioka, Soji**
**10-505, Higashi-naruse 2-2**
**Isehara-shi, Kanagawa-ken (JP)**
Inventor: **Muto, Hiroaki**
**2-14-45, Gochi**
**Joetsu-shi, Niigata-ken (JP)**
Inventor: **Onda, Yoshiro**
**1-9-3, chuo-cho**
**Higashi-kurume-shi, Tokyo (JP)**
Inventor: **Araume, Kiyoshi**
**3-11-21, Gochi**
**Joetsu-shi, Niigata-ken (JP)**

(74) Representative: **Armengaud, Alain et al**
**Cabinet ARMENGAUD AINE 3, Avenue**
**Bugeaud**
**F-75116 Paris (FR)**

(54) **Toiletry preparation.**

(57)    The invention provides a water-based toiletry composition, such as shampoos, hand lotions, hand creams and the like, containing a surface active agent and a specific thickening agent in an aqueous medium. The thickening agent is a water-soluble non-ionic cellulose ether of which at least a part of the hydroxyl groups are replaced with 3-alkoxy-2-hydroxypropoxyl groups, of which the alkoxy group has 6 to 26 carbon atoms. As compared with conventional water-soluble non-ionic cellulose ethers such as alkyl cellulose, hydroxyalkyl cellulose and hydroxyalkyl alkyl cellulose, the modified cellulose ether can exhibit a remarkably high thickening effect so that the composition can be imparted with the desired viscosity or consistency by compounding with only a very small amount of the thickening agent.

EP 0 457 672 A1

Jouve, 18, rue Saint-Denis, 75001 PARIS

## BACKGROUND OF THE INVENTION

The present invention relates to a toiletry preparation or, more particularly, to a toiletry preparation used for hygienic or aesthetic treatment of human bodies, skins and hairs including hair shampoos, body shampoos, hair rinses, hair-styling gels, hand lotions, milky lotions, hand creams, face packs and the like in the form of a liquid or pasty composition.

It is a usual formulation that these toiletry preparations are compounded with a water-soluble polymeric material with an object of stabilization of emulsions and suspensions, thickening, moisturization, flowability improvement and so on. Various kinds of water-soluble polymers are known and widely used for the purpose in the prior art including water-soluble non-ionic cellulose ethers such as methyl cellulose, hydroxyethyl cellulose, hydroxypropyl methyl cellulose and the like and water-soluble synthetic ionic polymers such as carboxyvinyl polymers and the like.

These conventional water-soluble polymers are not always satisfactory in one or more respects as an ingredient in a toiletry preparation. For example, the above named water-soluble non-ionic cellulose ethers in general cannot exhibit a thickening effect high enough so that the toiletry preparation can be imparted with a desired viscosity or consistency only by compounding a quite large amount of the polymer resulting in a rather unpleasant feeling of use given to the users if not to mention the disadvantageous increase in the manufacturing cost by using such a large amount of the polymer. The carboxyvinyl polymers, on the other hand, are excellent at least in the effect of thickening but a problem in the use of them is that the powder of the polymer can be uniformly dissolved in the aqueous medium only with some difficulties sometimes leaving undissolved lumps. In addition, the thickening effect of carboxyvinyl polymers can be fully exhibited only by the exact control of the pH value by the addition of a neutralizing agent such as sodium hydroxide, ammonia water, triethanol amine and the like so that manufacturing process of the toiletry preparation is rather complicated and sometimes accompanied by insufficient exhibition of the viscosity or consistency due to inaccuracy in the control of the pH value. In addition, the ionic nature of the carboxyvinyl polymers is the reason for the poor incompatibility of the polymer with electrolyte additives or ionic surface active agents so that the formulation of toiletry preparations using a carboxyvinyl polymer is greatly limited relative to the selection of other ingredients in this regard.

Japanese Patent Kokai 55-110103 teaches that toiletry preparations can be imparted with a high thickening effect by compounding with a specific water-soluble modified cellulose ether having long-chain alkyl groups of 10 to 24 carbon atoms introduced into a conventional water-soluble non-ionic cellulose ether by the reaction with a modifying agent such as long-chain alkyl epoxides, long-chain alkyl halides, long-chain alkyl isocyanates and the like. Modified cellulose ethers of this type, however, are not quite feasible as a thickening agent in toiletry preparations because of the expensiveness and low availability as a material for industrial use and susceptibility of the modified polymer to hydrolysis as well as corrosiveness of some of these modified polymers to decrease the durability of apparatuses.

## SUMMARY OF THE INVENTION

The present invention accordingly has an object to provide a novel water-based toiletry preparation compounded with a water-soluble polymer without being accompanied by the disadvantages and problems due to the inappropriateness of the water-soluble polymer compounded therein. Namely, the invention has an object to provide a toiletry preparation which is easy to prepare, is imparted with an adequately high viscosity or consistency by the addition of a relatively small amount of a water-soluble polymer as a thickening agent, has excellent stability in storage for a long period of time and gives pleasant feeling of use to the users.

Thus, the toiletry preparation of the invention is a composition which comprises, as a blend:

(a) an aqueous medium mainly consisting of water;

(b) from 0.1 to 20% by weight of a surface active agent dissolved in the aqueous medium; and

(c) from 0.01 to 20% by weight of a modified water-soluble non-ionic cellulose ether selected from the group consisting of alkyl celluloses, hydroxyalkyl celluloses and hydroxyalkyl alkyl celluloses, of which the alkyl group has 1 to 3 carbon atoms, hydroxyalkyl group has 2 to 4 carbon atoms and at least a part of the hydroxy groups are substituted by 3-alkoxy-2-hydroxypropoxyl groups represented by the general formula

$$R-O-CH_2-CHOH-CH_2-O-, \qquad (I)$$

in which R is an alkyl group having 6 to 26 carbon atoms, as a thickening agent dissolved in the aqueous medium, the balance being the aqueous medium and optional additives.

## DETAILED DESCRIPTION OF THE PREFERRED EMBODIMENTS

As is described above, the essential ingredients in the inventive toiletry preparation are an aqueous medium as the component (a), a surface active agent as the component (b) and a specific thickening agent as the component (c), of which the component (c) is the most characteristic of the inventive composition.

The modified water-soluble non-ionic cellulose ether as the component (c) is characterized by the specific modifying groups represented by the above given formula (I). Such a modified cellulose ether, which is a known polymeric material, can be prepared, for example, by the reaction of a starting cellulose ether such as alkyl celluloses, hydroxyalkyl celluloses and hydroxyalkyl alkyl celluloses, of which the alkyl group has 1 to 3 carbon atoms and hydroxyalkyl group has 2 to 4 carbon atoms, with a long-chain alkyl glycidyl ether, of which the long-chain alkyl group has 6 to 26 carbon atoms, so as to substitute the substituent groups of the formula (I) for at least a part of the hydroxyl groups.

Examples of the water-soluble non-ionic cellulose ether to be reacted with the long-chain alkyl glycidyl ether include methyl cellulose, hydroxyethyl cellulose, hydroxypropyl cellulose, hydroxyethyl methyl cellulose, hydroxypropyl methyl cellulose, hydroxyethyl ethyl cellulose and the like. The average degree of polymerization of these cellulose ethers is not particularly limitative but should preferably correspond to the viscosity of a 2% by weight aqueous solution of the cellulose ether in the range from 3 to 1,000,000 centipoise or, more preferably, from 200 to 300,000 centipoise at 20 °C. When the average degree of polymerization of the starting cellulose ether is too low, the resultant modified cellulose ether would be less effective as a thickening agent to impart the toiletry preparation with a desired viscosity or consistency. When the average degree of polymerization of the starting cellulose ether is too high, on the other hand, the starting cellulose ether would be less reactive with the long-chain alkyl glycidyl ether as the modifying agent.

The alkyl glycidyl ether as the modifying agent of the above described cellulose ether has a long-chain alkyl group having 6 to 26 carbon atoms as exemplified by stearyl glycidyl ether, lauryl glycidyl ether, cetyl glycidyl ether, decyl glycidyl ether, hexyl glycidyl ether, lignoceryl glycidyl ether, cerotyl glycidyl ether and the like though not particularly limitative thereto.

The reaction of the cellulose ether with the alkyl glycidyl ether is performed by first dispersing the cellulose ether in an organic solvent such as an aliphatic lower alcohol with addition of small amounts of water and an alkali such as alkali hydroxides to effect swelling of the cellulose ether followed by the addition of the alkyl glycidyl ether and agitation of the mixture at an elevated temperature to effect the reaction. After completion of the reaction, the residual alkali in the reaction mixture is neutralized and the product is purified and dried.

The amount of the alkyl glycidyl ether added to the reaction mixture depends on the desired degree of modification. It is usually preferble that the content of the modifying groups of the formula (I) in the modified cellulose ether is in the range from 0.05 to 10% by weight. When the degree of modification is too low, the modified cellulose ether cannot fully exhibit the thickening effect as desired. When the degree of modification is too high, on the other hand, the modified cellulose ether would be less soluble in the aqueous medium so that some difficulties are encountered in the manufacture of the toiletry preparation in addition to a decrease in the thickening effect. The content of the modifying groups of the formula (I) in the modified cellulsoe ether can be quantitatively determined by decomposing the modified cellulose ether with hydro-iodic acid and gas-chromatographically analyzing the alkyl iodide as the decomposition product in a xylene extract.

The modified cellulose ether as the component (c) prepared in the above described manner is dissolved or dispersed in an aqueous medium together with a surface active agent and other additives depending on the particular types of the toiletry prparations. The amount of the modified cellulose ether as the component (c) in the composition is usually in the range from 0.01 to 20% by weight or, in most cases, from 0.05 to 5% by weight though dependent on the particular type of the product. When the amount of the modified cellulose ether is too small, the desired effect of thickening cannot be fully obtained as a matter of course. When the amount thereof is too large, on the other hand, the composition would be too thick to be smoothly applied to and spread over the human body to give an unpleasant feeling of use to the user.

Although the medium of the inventive toiletry preparation consists basically of water, it is optional that the aqueous medium contains a small amount of a water-miscible organic solvent such as lower aliphatic alcohols, e.g., ethyl alcohol and isopropyl alcohol, and polyhydric alcohols, e.g., glycerin, propylene glycol and polyethylene glycol, provided that the solubility of the modified cellulose ether in the medium is not unduly decreased.

The toiletry preparations of the invention include various types of compositions having a viscosity or consistency ranging from a liquid to a paste used as a detergent such as hair shampoos and body shampoos, hair-conditiong agent such as hair rinses and hair-styling gels, toilet lotion such as hand lotions and milky lotions and cream such as hand creams as well as face packs. These toiletry preparations usually contain a surface active agent as an essential ingredient. The surface active agents formulated in these toiletry preparations include anionic surface active agents such as potassium salts of fatty acids, triethanolamine lauryl sulfate and sodium polyoxyethylene lauryl ether sulfates, non-ionic surface active agents such as fatty acid diethanolamides and beef tallow alkyl dimethylamine oxides and amphoteric surface active agents such as dimethyl alkyl betaines used in hair shampoos and body shampoos and long-chain alkyl quaternary ammonium salts as a cationic surface active agent such as diethyl dodecyl benzyl ammonium chloride and cetyl trimethyl ammonium chloride used in hair rinses. The amount of these surface active agents in the toiletry composition is usually in the range from 0.1 to 20% by weight.

The kinds and amounts of the other optional additives in the inventive toiletry preparations naturally depend on the types of the composition. Examples of such optional additive ingredients include pearlescent agents such as ethylene glycol distearate, fine mica flakes, comminuted fish scales and the like, turbidity agents such as calcium carbonate, titanium dioxide and the like, dandruff inhibitors such as zinc pyrithione and the like, antiseptic agents such as methylparaben and the like, pH controlling agents such as citric acid and the like, perfumes, dyes and others.

In the following, the toiletry preparation of the invention is described in more detail by way of examples and comparative examples, which, however, never limit the scope of the invention in any way, as preceded by the description of the preparation procedure of the modified cellulose ether as the component (c). The term of "parts" in the following description always refers to "parts by weight".

Synthetic Preparation 1.

A dispersion of 40 g of a hydroxypropyl methyl cellulose, of which a 2% by weight aqueous solution had a viscosity of 4200 centipoise at 20 °C (Metolose 60SH-4000, a product by Shin-Etsu Chemical Co.) in 400 g of tert-butyl alcohol was admixed with 35 g of a 6% by weight aqueous solution of sodium hydroxide and the mixture was agitated for 1 hour at room temperature under an atmosphere of nitrogen. Thereafter, 8 g of stearyl glycidyl ether (Epiol SK, a product by Nippon Oils and Fats Co.) were added to the mixture which was agitated for further 5 hours at 80 °C to effect the reaction. After the end of this reaction time, the reaction mixture was neutralized with addition of a small amount of acetic acid and, after cooling to room temperature, the mixture was filtered to recover the solid material therein which was repeatedly washed first twice each time with 10 times by volume of hexane, then twice each time with 10 times by volume of acetone and finally with 100 times by volume of hot water followed by drying to give a modified cellulose ether product. The total content of 3-octadecyloxy-2-hydroxypropoxy groups and 3-hexadecyloxy-2-hydroxypropoxy groups in the product was 0.60% by weight and a 2% by weight aqueous solution thereof had a viscosity of 150,000 centipoise at 20 °C. This product is referred to as the modified cellulose ether I hereinbelow.

Synthetic Preparation 2.

The synthetic procedure was substantially the same as in Synthetic Preparation 1 described above except that the hydroxypropyl methyl cellulose was replaced with the same amount of a methyl cellulose, of which a 2% by weight aqueous solution had a viscosity of 4100 centipoise at 20 °C (Metolose SM-4000, a product by Shin-Etsu Chemical Co.) and the amount of the stearyl glycidyl ether was decreased to 5 g. The total content of 3-octadecyloxy-2-hydroxypropoxy groups and 3-hexadecyloxy-2-hydroxypropoxy groups in the product was 0.35% by weight and a 2% by weight aqueous solution thereof had a viscosity of 110,000 centipoise at 20 °C. This product is referred to as the modified cellulose ether II hereinbelow.

Synthetic Preparation 3.

The synthetic procedure was substantially the same as in Synthetic Preparation 2 described above except that the stearyl glycidyl ether was replaced with the same amount of decyl glycidyl ether (Epiol L-41, a product by Nippon Oils and Fats Co.). The content of 3-decyloxy-2-hydroxypropyl groups in the product was 0.48% by weight and a 2% by weight aqueous solution thereof had a viscosity of 120,000 centipoise at 20 °C. This product is referred to as the modified cellulose ether III hereinbelow.

Synthetic Preparation 4.

The synthetic procedure was substantially the same as in Synthetic Preparation 1 described above except that the hydroxypropyl methyl cellulose Metolose 60SH-4000 was replaced with the same amount of another hydroxypropyl methyl cellulose, of which a 2% by weight aqueous solution had a viscosity of 70,000 centipoise at 20 °C and the contents of the methoxyl groups and hydroxypropoxyl groups were 24% by weight and 6.4% by weight, respectively (Metolose 90SH-10000, a product by Shin-Etsu Chemical Co.), and the length of time of the reaction at 80 °C was 10 hours instead of 5 hours. The total content of 3-octadecyloxy-2-hydroxypropoxyl groups and 3-hexadecyloxy-2-hydroxypropoxyl groups in the product was 0.65% by weight and a 2% by weight solution thereof in a 5:1 by weight mixture of water and isopropyl alcohol had a viscosity of 105,000 centipoise at 20 °C. This product is referred to as the modified cellulose ether IV hereinbelow.

Synthetic Preparation 5.

The synthetic procedure was substantially the same as in Synthetic Preparation 4 described above except that the amount of the stearyl glycidyl ether was increased to 15 g. The total content of 3-octadecyloxy-2-hydroxypropoxyl groups and 3-hexadecyloxy-2-hydroxypropoxyl groups in the product was 1.73% by weight and a 2% by weight solution thereof in a 5:1 by weight mix-ture of water and isopropyl alcohol had a viscosity of 145,000 centipoise at 20 °C. This product is referred to as the modified cellulose ether V hereinbelow.

## Synthetic Preparation 6.

The synthetic procedure was substantially the same as in Synthetic Preparation 3 described above except that the hydroxypropyl methyl cellulose was replaced with the same amount of a hydroxyethyl methyl cellulose, of which a 2% by weight aqueous solution had a viscosity of 32,000 centipoise at 20 °C (Metolose 60SEW-30T, a product by Shin-Etsu Chemical Co.), and the length of time of the reaction at 80 °C was 10 hours instead of 5 hours. The content of 3-decyloxy-2-hydroxypropoxyl groups in the product was 1.02% by weight and a 2% by weight solution thereof in a 5:1 by weight mixture of water and isopropyl alcohol had a viscosity of 98,000 centipoise at 20 °C. This product is referred to as the modified cellulose ether VI hereinbelow.

## Example 1.

Three hair shampoo compositions were prepared each in a conventional manner from 30 parts of a 2% by weight aqueous solution of one of the modified cellulose ethers I, II and III prepared above, 40 parts of a 35% by weight aqueous solution of a sodium polyoxyethylene lauryl sulfate, 5 parts of lauric acid ethanolamide, 1 part of zinc pyrithione and 0.2 part of menthol, the balance to make up a total amount of 100 parts being water. These hair shampoo compositions had viscosities of 3200 centipoise, 4000 centipoise and 4500 centipoise, respectively, at 20 °C.

For comparison, another hair shampoo composition was prepared in the same formulation as above except that the modified cellulose ether was replaced with the same amount of the hydroxypropyl methyl cellulose, which was the cellulose ether used as the starting material in Synthetic Preparation 1, before modification. The thus prepared comparative hair shampoo composition had a viscosity of only 320 centipoise at 20 °C.

To examine the stability of the dispersion of zinc pyrithione in the shampoo composition, the above prepared four hair shampoo compositions were kept standing for 1 month at 40 °C to give the results that precipitates were found in the comparative shampoo composition while absolutely no precipitates were found in each of the three shampoo compositions prepared from the modified cellulose ethers.

## Example 2.

Three hair rinse compositions were prepared each in a conventional manner from 10 parts of a 2% by weight aqueous solution of one of the modified cellulose ethers I, II and III prepared above, 3 parts of cetyl trimethyl ammonium chloride, 2 parts of cetyl alcohol, 6 parts of propylene glycol and 0.2 part of menthol, the balance to make up a total amount of 100 parts being water. These hair rinse compositions had viscosities of 1200 centipoise, 960 centipoise and 1100 centipoise, respectively, at 20 °C.

For comparison, another hair rinse composition was prepared in the same formulation as above except that the modified cellulose ether was replaced with the same amount of the hydroxypropyl methyl cellulose, which was the cellulose ether used as the starting material in Synthetic Preparation 1, before modification. The thus prepared comparative hair rinse composition had a viscosity of only 320 centipoise at 20 °C.

## Example 3.

Three body shampoo compositions were prepared each in a conventional manner from 10 parts of a 2% by weight aqueous solution of one of the modified cellulose ethers I, II and III prepared above, 15 parts of a potassium salt of cocofatty acid, 10 parts of cocoamidopropyl betaine and 0.2 part of menthol, the balance to make up a total amount of 100 parts being water. These body shampoo compositions had viscosities of 1150 centipoise, 900 centipoise and 1000 centipoise, respectively, at 20 °C.

For comparison, another body shampoo composition was prepared in the same formulation as above except that the modified cellulose ether was replaced with the same amount of the hydroxypropyl methyl cellulose, which was the cellulose ether used as the starting material in Synthetic Preparation 1, before modification. The thus prepared comparative body shampoo composition had a viscosity of only 300 centipoise at 20 °C.

## Example 4.

A hair-styling gel composition was prepared by first dispersing 2.0 parts of the modified cellulose ether IV in 87.0 parts of water kept at 70 °C with agitation followed by the addition of 1.0 part of 1,3-butylene glycol, 0.5 part of polyoxyethylene monostearate and 10.0 parts of ethyl alcohol and cooling to room temperature. The thus prepared gel composition was very uniform absolutely without undissolved lumps such as those sometimes unavoidably formed in a similar preparation by using a carboxyvinyl polymer as the gelling agent. The product could give a very pleasant feeling of use to the users.

## Example 5.

A wipe-off face pack composition was prepared from 2.0 parts of the modified cellulose ether VI, 1.0 part of polyoxyethylene oleyl ether, 5.0 parts of ethyl alcohol and 92.0 parts of water. This face pack composition could give a pleasant feeling of use to the users.

Example 6.

Two hand lotion compositions were prepared each from 0.5 part of the modified cellulose ether V or VI, 5 parts of propylene glycol, 1.5 parts of polyoxyethylene oleyl ether, 10 parts of ethyl alcohol and 83 parts of water. These hand lotion compositions had viscosities of 1300 centipoise and 750 centipoise, respectively, at 20 °C.

For comparison, two more hand lotion compositions were prepared each in the same formulation as above except that the modified cellulose ether was replaced with the same amount of the unmodified cellulose ether which was the starting cellulose ether used in the prepration of the modified cellulose ethers V or VI, respectively. These comparative hand lotion compositions had viscosities of 410 centipoise and 230 centipoise, respectively, at 20 °C.

Example 7.

A milky lotion was prepared from 1.5 parts of stearic acid, 0.5 part of cetyl alcohol, 5.0 parts of propylene glycol, 10.0 parts of liquid paraffin, 2.0 parts of polyoxyethylene oleyl ether, 0.5 part of the modified cellulose ether V and water in an amount to make up a total amount of 100 parts. The thus prepared milky lotion was very stable and could give a pleasant feeling of use to the users.

Example 8.

A hand cream composition was prepared from 1.5 parts of stearic acid, 0.5 part of cetyl alcohol, 5.0 parts of propylene glycol, 10.0 parts of liquid paraffin, 2.0 parts of polyoxyethylene oleyl ether, 1.5 parts of the modified cellulose ether V and 79.5 parts of water. The thus prepared hand cream composition had a viscosity of 142,500 centipoise at 20 °C.

For comparison, another hand cream composition was prepared in the same formulation as above except that the modified cellulose ether V was replaced with the same amount of the unmodified cellulose ether which was the starting cellulose ether used in the preparation of the modified cellulose ether V. This comparative hand cream composition had a viscosity of 36,500 centipoise at 20 °C.

## Claims

1. A toiletry preparation which comprises, as a blend:

(a) an aqueous medium mainly consisting of water;

(b) a surface active agent dissolved in the aqueous medium; and

(c) a modified water-soluble cellulose ether selected from the group consisting of alkyl celluloses, hydroxyalkyl celluloses and hydroxyalkyl alkyl celluloses, of which the alkyl group has 1 to 3 carbon atoms, hydroxyalkyl group has 2 to 4 carbon atoms and at least a part of the hydroxy groups are substituted by 3-alkoxy-2-hydroxypropoxyl groups represented by the general formula

$$R-O-CH_2-CHOH-CH_2-O-,$$

in which R is an alkyl group having 6 to 26 carbon atoms, as a thickening agent dissolved in the aqueous medium.

2. The toiletry preparation as claimed in claim 1 in which the amount of the surface active agent is in the range from 0.1 to 20% by weight.

3. The toiletry preparation as claimed in claim 1 in which the amount of the modified water-soluble non-ionic cellulose ether is in the range from 0.01 to 20% by weight.

4. The toiletry preparation as claimed in claim 1 in which the alkyl group having 6 to 26 carbon atoms denoted by R is selected from the class consisting of stearyl, lauryl, cetyl, decyl, hexyl, lignoceryl, cerotyl and hexadodecyl groups.

5. The toiletry preparation as claimed in claim 1 in which the modified water-soluble cellulose ether contains from 0.05 to 10% by weight of the 3-alkoxy-2-hydroxypropoxyl groups.

EP 0 457 672 A1

European Patent Office

**EUROPEAN SEARCH REPORT**

EP 91 40 1244

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 213 527 (UNION CARBIDE CORP.) * Page 4, line 1 - page 5, line 2; page 8, lines 11-19; page 17, line 17 - page 14, line 12; claim 1 * --- | 1-5 | A 61 K 7/06 A 61 K 7/48 C 11 D 3/22 |
| Y | EP-A-0 362 769 (AQUALON CO.) * Page 2, line 1 - page 3, line 38; claims 1,10 * --- | 1-5 | |
| P,A | PATENT ABSTRACTS OF JAPAN, vol. 15, no. 128 (C-818)[4656], 28th March 1991; & JP-A-3 12 401 (SHIN ETSU CHEM. CO., LTD) 21-01-1991 * Whole abstract * ----- | 1 | |

| | | | TECHNICAL FIELDS SEARCHED (Int. Cl.5) |
|---|---|---|---|
| | | | A 61 K C 11 D |

The present search report has been drawn up for all claims

| Place of search | Date of completion of the search | Examiner |
|---|---|---|
| THE HAGUE | 07-08-1991 | COUCKUYT P.J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

7